# EUROPEAN PATENT APPLICATION

(11) **EP 4 563 101 A1**
(43) Date of publication of application: **04.06.2025**
(21) Application number: 23851714.8
(22) Date of filing: 04.08.2023
(51) Int. Cl.: A61B 17/29

(54) **MINIMALLY INVASIVE SURGICAL INSTRUMENT ASSEMBLY**

(30) Priority: 09.08.2022 CN 202210951861
(71) Applicant: CTC Medical Technology (Beijing) Co., Ltd, Beijing 100176 (CN); Qingda CTC (Xiamen) Medical Technology Co., Ltd, Xiamen, Fujian 361101 (CN)
(72) Inventor: WEI, Wei, Beijing 100176 (CN); JIANG, Peng, Beijing 100176 (CN); SHAO, Meng, Beijing 100176 (CN); ZHAO, Lei, Beijing 100176 (CN); SUN, Dawei, Beijing 100176 (CN); ZHOU, Xu, Beijing 100176 (CN); LI, Xiaoming, Beijing 100176 (CN)
(74) Representative: Derry, Paul Stefan
(86) International application number: PCT/CN2023/111210
(87) International publication number: WO 2024/032493

(57) **Abstract**

The present disclosure relates to the field of surgical instruments, and discloses a minimally invasive surgical instrument assembly and a minimally invasive surgical instrument. The minimally invasive surgical instrument assembly includes a rotatable wrist designed for attaching an end effector. The rotatable wrist is hinged to a fixed rod, with a linkage hinged at one end to the rotatable wrist and at the other end to a first retractable rod. This configuration allows the rotatable wrist to be driven to rotate about a first hinge when the first retractable rod moves retractably along the longitudinal length of the fixed rod, thereby enabling the rotation of the end effector. Moreover, the disclosed structure features a smaller turning radius compared to existing joint wrist structures, effectively addressing challenges faced by minimally invasive surgical instruments, such as difficulties in rotating the end effector within confined spaces or potential interference with other surgical instruments due to their larger turning radius.

## Description

### Technical Field

The present disclosure relates to the field of surgical instruments, and in particularly to a minimally invasive surgical instrument assembly and a minimally invasive surgical instrument.

### Background

Minimally invasive surgery involves forming one or several small incisions, typically 5-12 millimeters in diameter, in the abdomen of a human body, inserting an endoscope and various specialized surgical instruments through these small incisions, capturing images of various visceral organs in the abdominal cavity with the endoscope inserted into the abdomen and transmitting these images to a monitor screen, and enabling a surgeon to use the surgical instruments to perform operations in vitro by observing the images.

Since minimally invasive surgical instruments are inserted into the body while operations are performed externally, their structure is characterized by an elongated rod body for insertion into the human body, attached to an operating handle, with a movable end effector arranged at an end of the rod body. The position of the end effector in the human body must be adjusted according to surgical requirements, hence existing minimally invasive surgical instruments feature a multi-joint wrist structure located in the middle of the rod body. This structure enables rotation of the end effector and the rod body section connected to it by adjusting the multi-joint wrist. However, the multi-joint wrist structure, due to its length, has a relatively large turning radius. This becomes problematic when the space at a lesion site is narrow or when multiple surgical instruments are inserted for simultaneous operation. In such cases, existing minimally invasive surgical instruments may be unable to rotate the end effector within the confined space or may interfere with other instruments due to their large turning radius.

### Summary

The main disadvantage of conventional technologies is that existing minimally invasive surgical instruments with a multi-joint wrist structure in the middle of the rod body cannot allow the end effector to rotate in narrow spaces or interfere with other surgical instruments due to their relatively large turning radius. Accordingly, there is a need in the art for an improved minimally invasive surgical instrument assembly and minimally invasive surgical instrument.

To solve the above problems, an aspect of the disclosure provides a minimally invasive surgical instrument assembly, which may include:
a rotatable wrist with a distal end to which an end effector is attachable and a proximal end provided with a first connecting part and a second connecting part;
a fixed rod with a distal end rotatably connected to the first connecting part via a first hinge;
a linkage with a distal end rotatably hinged to the second connecting part; and
a first retractable rod detachably connected to the fixed rod, with a distal end rotatably hinged to a proximal end of the linkage, the first retractable rod being drivable to move retractably along a longitudinal length of the fixed rod, to force the rotatable wrist to rotate around the first hinge relative to the fixed rod.

Optionally, the fixed rod may have a tubular structure with the first hinge mounting base at its distal end, where the first hinge mounting base may be hinged to the first connecting part, and the tube wall of the fixed rod may be provided with a first receiving groove communicating with an internal space of the tubular structure, the first retractable rod may be located inside the fixed rod, and the distal end of the first retractable rod may be received in the first receiving groove to connect with the proximal end of the linkage.

Optionally, the fixed rod may include a fixed tube and a fixed tube connector that may be detachably connected, where the fixed tube may have a straight tubular structure, and the first hinge mounting base and the first receiving groove may be arranged distally on the fixed tube connector.

Optionally, an L-shaped groove arranged in a tube wall at a distal end of the fixed tube may include a limit portion extending circumferentially around the fixed tube and an access passage extending axially along the fixed tube, where the access passage may defines an opening at an end of the fixed tube, and a radially protruding limit protrusion may be arranged on an outer peripheral surface of a proximal end of the fixed tube connector, where the limit protrusion may be engageable or disengageable from the limit portion through the access passage, and the limit portion may be adapted in length to the limit protrusion along the axial direction of the fixed tube.

Optionally, the proximal end of the rotatable wrist may include a tube wall with an axially protruded portion forming the first connecting part and a non-protruded portion forming the second connecting part.

Optionally, the rotatable wrist may be provided with a space for sliding.

The minimally invasive surgical instrument assembly may further include:
an end effector with an installation end located within the space for sliding;
a flexibly deformable connector positioned where the rotatable wrist may be connected to the fixed rod, with a distal end attached to the installation end of the end effector; and
a second retractable rod with a distal end connected to a proximal end of the connector, where the second retractable rod may be drivable to move retractably along the longitudinal length of the fixed rod, to force the end effector to move retractably in a direction of extending into or retracting from the space for sliding.

Optionally, the fixed rod may have a tubular structure with a first hinge mounting base at its distal end, where the second retractable rod may be located inside the fixed rod, and an opening for the connector to pass through may be arranged between the first hinge mounting base and a tube wall of the fixed rod.

Additionally, the rotatable wrist may be internally hollow to form the space for sliding and a channel for the connector to enter the space for sliding.

Optionally, the first hinge mounting base may be provided with a limit bar, which may be arranged radially along the fixed rod, and a limit space adapted for the connector to pass through may be formed between the limit bar and the first connecting part.

Additionally or alternatively, the connector may extend from a centerline of the rotatable wrist to a centerline of the fixed rod.

Optionally, the first retractable rod may have a tubular structure with a second hinge mounting base at its distal end, where the second hinge mounting base may be hinged to the proximal end of the linkage, the first retractable rod may be coaxially sleeved outside the second retractable rod, and the second retractable rod may be drivable to move retractably relative to the fixed rod.

Optionally, the first retractable rod may include a first retractable rod tube and a first retractable rod tube connector that may be detachably connected, where the first retractable rod tube may have a straight tubular structure, the second hinge mounting base may be arranged distally on the first retractable rod tube connector, and the first retractable rod tube connector may be further provided with an opening for the second retractable rod or the connector to pass through.

Optionally, the first retractable rod tube may have a tube wall provided with two bayonets, and the first retractable rod tube connector may have a proximal end provided with two elastic hooks, each capable of extending into the first retractable rod tube to engage with one of the two bayonets respectively, where the opening for the second retractable rod or the connector to pass through may be positioned between the two elastic hooks.

Optionally, the second retractable rod may include a second retractable rod body located inside the first retractable rod tube and a second retractable rod body connector, with its distal end positioned between the second hinge mounting base and a tube wall of the fixed tube connector of the fixed rod, and its proximal end passing through the opening in the first retractable rod tube connector and being detachably connected to the first retractable rod tube.

Optionally, the connector may be an elastic sheet, an elastic rod or a flexible rope.

Optionally, the end effector may be a clamp that may be driven by the second retractable rod to open and close, where the clamp may include:
two claws, at least one of which may be provided with an arcuate limit hole and may be rotatably connected to the rotatable wrist via a first rotating structure, where the first rotating structure may have an axis of rotation perpendicular to a direction of movement of the end effector, and the first rotating structure may be positioned on a concave curved side of the arcuate limit hole;
a claw mounting base provided with a linear limit hole extending along a direction of extension and retraction of the end effector, and provided with an abutment for abutting against the clamp provided with the arcuate limit hole on a side of the linear limit hole facing the fixed rod, where the claw mounting base may be connected to the connector; and
a limit pin arranged at the rotatable wrist, where the limit pin may penetrate through the linear limit hole and the arcuate limit hole.

Optionally, the clamp provided with the arcuate limit hole may be rotatably connected to the claw mounting base via a second rotating structure, where the second rotating structure may have an axis of rotation perpendicular to the direction of movement of the end effector, and the second rotating structure may be positioned on a side of the arcuate limit hole different from the concave curved side of the arcuate limit hole.

Another aspect of the disclosure provides a minimally invasive surgical instrument, which may include the described minimally invasive surgical instrument assembly.

By utilizing the disclosed solutions, the rotatable wrist designed for attaching an end effector is hinged to the fixed rod, with the linkage hinged at one end to the rotatable wrist and at the other end to the first retractable rod. This configuration allows the rotatable wrist to be driven to rotate about the first hinge when the first retractable rod moves retractably along the longitudinal length of the fixed rod, thereby enabling the rotation of the end effector. Moreover, the disclosed structure features the smaller turning radius compared to existing joint wrist structures, effectively addressing challenges faced by minimally invasive surgical instruments, such as difficulties in rotating the end effector within confined spaces or potential interference with other surgical instruments due to their larger turning radius.

### Brief Description of the Drawings

Fig. 1 is a front view of an embodiment of a minimally invasive surgical instrument provided by the disclosure;
Fig. 2 is a structural schematic view of the minimally invasive surgical instrument in Fig. 1;
Fig. 3 is a sectional view of a first embodiment of a minimally invasive surgical instrument assembly provided by the disclosure;
Fig. 4 is a bottom view of the minimally invasive surgical instrument assembly in Fig. 3;
Fig. 5 is a sectional view of a second embodiment of a minimally invasive surgical instrument assembly provided by the disclosure;
Fig. 6 is a sectional view of a third embodiment of a minimally invasive surgical instrument assembly provided by the disclosure;
Fig. 7 is a structural schematic view of the minimally invasive surgical instrument assembly in Fig. 6;
Fig. 8 is a structural schematic view of a connector in Fig. 3;
Fig. 9 is a structural schematic view of a distal end of a fixed rod in the disclosure;
Fig. 10 is a structural schematic view of a fixed tube in Fig. 9;
Fig. 11 is a structural schematic view of a first embodiment of a fixed tube connector in Fig. 9;
Fig. 12 is a structural schematic view of a second embodiment of the fixed tube connector in Fig. 9;
Fig. 13 is a structural schematic view of a proximal end of a fixed rod in the disclosure;
Fig. 14 is a structural schematic view of a distal end of a first retractable rod in the disclosure;
Fig. 15 is a structural schematic view of a first retractable rod tube connector in Fig. 14;
Fig. 16 is a structural schematic view of a first retractable rod tube in Fig. 14;
Fig. 17 is a left view of Fig. 16;
Fig. 18 is an exploded view of an end effector in the disclosure;
Fig. 19 is a front view of a first embodiment of a quick releaser in the disclosure;
Fig. 20 is a front sectional view of the quick releaser in Fig. 19;
Fig. 21 is a structural schematic view of a second embodiment of the quick releaser in the disclosure;
Fig. 22 is a front sectional view of the quick releaser in Fig. 21;
Fig. 23 is a front sectional view of a third embodiment of a quick releaser in the disclosure;
Fig. 24 is a structural schematic view of a part of the quick releaser in Fig. 23;
Fig. 25 is a front sectional view of a proximal end of the minimally invasive surgical instrument in Fig. 1;
Fig. 26 is a front sectional view of proximal ends of a fixed rod and a first retractable rod in Fig. 25;
Fig. 27 is a structural schematic view of a rotating thumbwheel in Fig. 25;
Fig. 28 is a schematic view of installation of a first trigger in Fig. 25;
Fig. 29 is a structural schematic view of the first trigger in Fig. 25;
Fig. 30 is a structural schematic view of a second trigger in Fig. 25;
Fig. 31 is a top view of the second trigger in Fig. 30; and
Fig. 32 is a schematic exploded view of a minimally invasive surgical instrument assembly provided by the disclosure in a cleaning state.

### Detailed Description

Embodiments of the present disclosure will be illustrated in detail hereinafter with reference to the accompanying drawings. It should be understood that the embodiments described herein are merely intended to illustrate and explain the disclosure, but not to impose any limitation on its scope.

In the disclosure, unless otherwise specified, the directional terms "proximal/proximally" and "distal/distally" are defined relative to a position of a hand performing the holding operation when the minimally invasive surgical instrument is in use. Specifically, an end closer to the hand is referred to as the proximal end, while an end farther from the hand is referred to as the distal end.

According to an aspect of the disclosure, a minimally invasive surgical instrument assembly includes a rotatable wrist 1, a fixed rod 5, a linkage 7, and a first retractable rod 8. The rotatable wrist 1 can be attached at its distal end to an end effector 2 and is provided at its proximal end with a first connecting part 3 and a second connecting part 4. The fixed rod 5 is rotatably connected at its distal end to the first connecting part 3 via a first hinge 6. The linkage 7 is rotatably hinged at its distal end to the second connecting part 4. The first retractable rod 8 is detachably connected to the fixed rod 5, with its distal end rotatably hinged to a proximal end of the linkage 7, and the first retractable rod 8 can be driven to move retractably along a longitudinal length of the fixed rod 5, to force the rotatable wrist 1 to rotate around the first hinge 6 relative to the fixed rod 5.

By utilizing the disclosed solutions, the rotatable wrist 1 designed for attaching an end effector 2 is hinged to the fixed rod 5, with the linkage 7 hinged at one end to the rotatable wrist 1 and at the other end to the first retractable rod 8. This configuration allows the rotatable wrist 1 to be driven to rotate about the first hinge 6 when the first retractable rod 8 moves retractably along the longitudinal length of the fixed rod 5, thereby enabling the rotation of the end effector 2. Moreover, the disclosed structure features the smaller turning radius compared to existing joint wrist structures, effectively addressing challenges faced by minimally invasive surgical instruments, such as difficulties in rotating the end effector 2 within confined spaces or potential interference with other surgical instruments due to their larger turning radius.

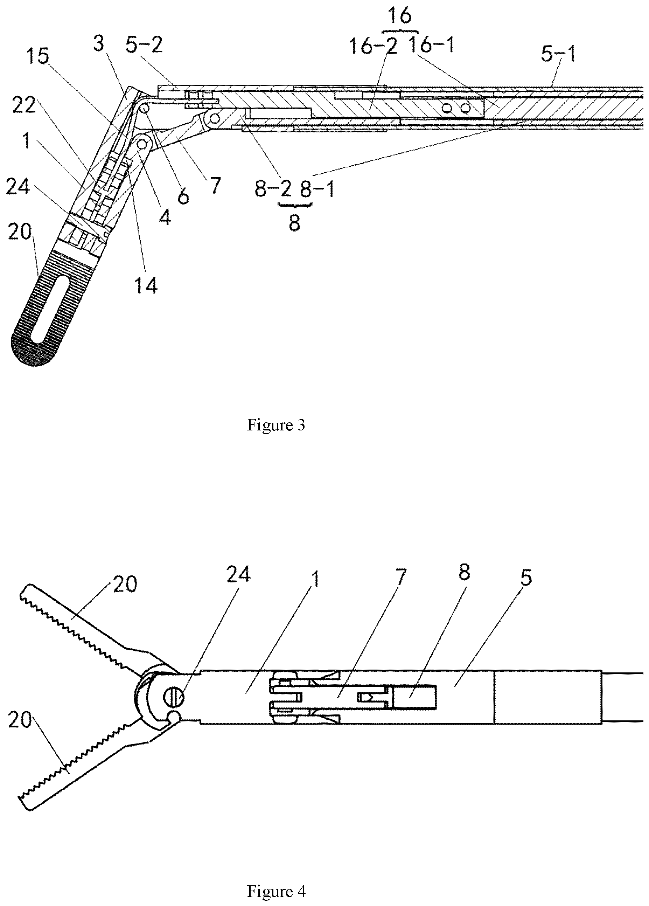

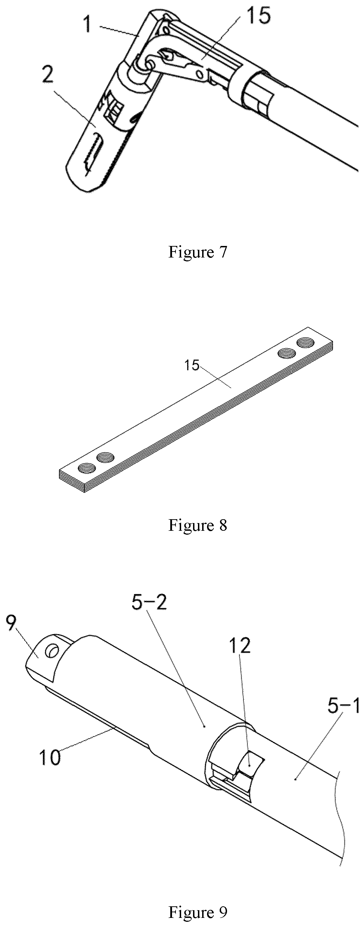

In an embodiment shown in Figs. 3 and 9, the fixed rod 5 has a tubular structure with a first hinge mounting base 9 at its distal end. In particularly, the first hinge mounting base 9 protrudes from a tube wall of the fixed rod 5 axially along the fixed rod 5. The first hinge mounting base 9 is hinged to the first connecting part 3, and the tube wall of the fixed rod 5 is provided with a first receiving groove 10 that communicates with an internal space of the tube. The first retractable rod 8 is located inside the fixed rod 5, with its distal end positioned inside the first receiving groove 10 to connect with the proximal end of the linkage 7. On one hand, the first receiving groove 10 serves to guide and limit the distal end of the first retractable rod 8, allowing it to move along the guidance of the first receiving groove 10. On the other hand, the first receiving groove 10 also accommodates the proximal end of the linkage 7. When the minimally invasive surgical instrument assembly is extended into or retracted from the human body, the end effector 2 and the rotatable wrist 1 remain aligned with the axis of the fixed rod 5, meaning they will not rotate. Now, the proximal end of the linkage 7 is positioned in the first receiving groove 10, such that the linkage 7 will not protrude from an outer peripheral wall of the fixed rod 5, which can prevent the linkage 7 from causing scratches or injury to the human body during the insertion or retraction of the minimally invasive surgical instrument assembly. However, in other embodiments, the fixed rod 5 may have a solid rod body structure, with the first hinge mounting base 9 positioned at its distal end, eliminating the need for the first receiving groove 10. In this configuration, the first retractable rod 8 is arranged parallel to the fixed rod 5. To ensure that the fixed rod 5 and the first retractable rod 8 can extend into or retract from the human body synchronously without causing scratches or injury to the human body during movement, a tube sleeve is provided to enclose both the fixed rod 5 and the first retractable rod 8 as a whole. Thus, both components are housed inside the tube sleeve.

In an embodiment shown in Figs. 9-12, the fixed rod 5 includes a fixed tube 5-1 and a fixed tube connector 5-2 that are detachably connected, where the fixed tube 5-1 has a straight tubular structure, and the first hinge mounting base 9 and the first receiving groove 10 are arranged distally on the fixed tube connector 5-2.

Since the fixed rod 5 needs to include the first hinge mounting base 9, it is designed as a detachable structure including the fixed tube 5-1 and the fixed tube connector 5-2. This design offers two advantages: first, it simplifies the production and manufacturing process, allowing the straight tubular fixed tube 5-1 and the specially shaped fixed tube connector 5-2 to be manufactured separately; second, it facilitates the disassembly and installation of the fixed rod 5. However, in other embodiments, the fixed rod 5 may also be an integrated structure, meaning that the fixed tube 5-1 and the fixed tube connector 5-2 are either formed as an integrated structure or fixedly connected.

In the embodiment shown in Figs. 9-12, an L-shaped groove 11 arranged in a tube wall at the distal end of the fixed tube 5-1 includes a limit portion extending circumferentially around the fixed tube 5-1 and an access passage extending axially along the fixed tube 5-1. The access passage defines an opening at the end of the fixed tube 5-1, and a radially protruding limit protrusion 12 is arranged on an outer peripheral surface at a proximal end of the fixed tube connector 5-2. The limit protrusion 12 can be engaged or disengaged from the limit portion through the access passage. The limit portion is adapted in length to the limit protrusion 12 in the axial direction of the fixed tube 5-1.

The detachable connection structure of the L-shaped groove 11 and the limit protrusion 12 can only restrict the axial positions of the fixed tube 5-1 and the fixed tube connector 5-2, but cannot restrict their circumferential positions. This means there can be relative rotation between the fixed tube 5-1 and the fixed tube connector 5-2 around a central axis of the fixed rod 5. In this embodiment, a rotating thumbwheel 31-2 is provided to rotate the first retractable rod 8 around its central axis, thereby driving the end effector 2 and the rotatable wrist 1 to rotate. As a result, when the first retractable rod 8 is rotated, the rotatable wrist 1 will drive the fixed tube connector 5-2 to rotate around the central axis of the fixed rod 5 relative to the fixed tube 5-1. This rotation introduces a risk of detachment between the fixed tube 5-1 and the fixed tube connector 5-2. Therefore, in this embodiment, the proximal end of the fixed rod 5, specifically the proximal end of the fixed tube 5-1, is connected to the first retractable rod 8 via a circumferential limit member 46. This configuration ensures the circumferential limitation of the first retractable rod 8 and the fixed tube 5-1 along the fixed rod 5, allowing the first retractable rod 8 to rotate synchronously with the fixed tube 5-1. As a result, the risk of detachment between the fixed tube 5-1 and the fixed tube connector 5-2 can be effectively eliminated. However, in other embodiments, the rotating thumbwheel 31-2 may be omitted, meaning the first retractable rod 8 and the fixed rod 5 would not rotate around the central axis of the fixed rod 5. In this case, the detachable connection structure of the L-shaped groove 11 and the limit protrusion 12 will not pose a risk of detachment between the fixed tube 5-1 and the fixed tube connector 5-2. However, in other embodiments, the fixed tube 5-1 and the fixed tube connector 5-2 may also be connected by using a detachable connection structure of bayonets 18 and elastic hooks 19, as described below.

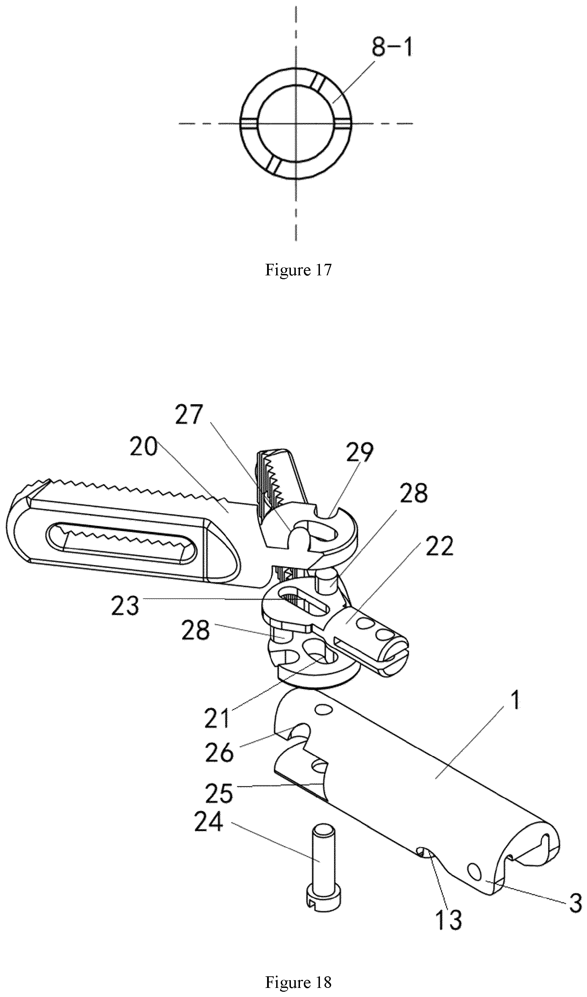

In an embodiment shown in Figs. 3 and 18, the tube wall at the proximal end of the rotatable wrist 1 includes a portion protrudes axially to form the first connecting part 3, and a non-protruded portion to form the second connecting part 4. At an axial position of the non-protruded portion of the tube wall, a second receiving groove 13 is formed on one side of the first connecting part 3. The second receiving groove 13 receives the distal end of the linkage 7, such that the linkage 7 will not protrude beyond an outer peripheral wall of the rotatable wrist 1. This configuration prevents the linkage 7 from causing scratches to the human body when the minimally invasive surgical instrument assembly is inserted into or withdrawn from the human body. In this embodiment, the outer peripheral wall of the rotatable wrist 1 aligns with the outer peripheral wall of the fixed rod 5 on the same circumferential surface. This alignment prevents the connection position between the rotatable wrist 1 and the fixed rod 5 from causing scratches to the human body when the minimally invasive surgical instrument assembly is inserted into or withdrawn from the body.

As shown in Figs. 3, 5, and 6, the rotatable wrist 1 is provided with a space for sliding 14. The minimally invasive surgical instrument assembly further includes the end effector 2, a flexibly deformable connector 15, and a second retractable rod 16. An installation end of the end effector 2 is positioned within the space for sliding 14. The connector 15 is located at a position where the rotatable wrist 1 and the fixed rod 5 are connected, with its distal end connected to the installation end of the end effector 2. The second retractable rod 16 is connected at its distal end to the proximal end of the connector 15. The second retractable rod 16 can be driven to move retractably along the longitudinal length of the fixed rod 5, to force the end effector 2 to move retractably in a direction of extending into or retracting from the space for sliding 14. In this embodiment, the space for sliding 14 is provided at the end effector 2, along with the connector 15 and the second retractable rod 16, which is driven to move retractably along the longitudinal length of the fixed rod 5, thereby enabling the end effector 2 to perform extensible and retractable movement guided by the space for sliding 14. During surgery, a depth of the minimally invasive surgical instrument assembly inserted into the human body is controlled manually to achieve coarse adjustment of a position of the end effector 2. Fine adjustment of the position of the end effector 2 is achieved through the extensible and retractable movement of the second retractable rod 16, enhancing operational convenience. Additionally, the extensible and retractable movement of the second retractable rod 16 drives the end effector 2, enabling it to perform scraping or cutting operations at a lesion site. In addition, the flexibly deformable connector 15 can adapt to a rotation angle of the end effector 2 by deforming accordingly. When the second retractable rod 16 drives a change in the position of the end effector 2, a bending deformation position of the connector 15 shifts as needed, ensuring a continuous connection between the end effector 2 and the second retractable rod 16. This flexibility allows the second retractable rod 16 to effectively move and control the end effector 2, even when the end effector 2 rotates at an angle. However, in other embodiments, the end effector 2 may also be fixedly attached to the rotatable wrist 1, eliminating the need for the second retractable rod 16.

In the embodiment shown in Figs. 3 and 9, the fixed rod 5 features a tubular structure with a first hinge mounting base 9 at its distal end. The second retractable rod 16 is positioned inside the fixed rod 5, and an opening is provided between the first hinge mounting base 9 and the tube wall of the fixed rod 5, allowing the connector 15 to pass through. The rotatable wrist 1 is hollow, forming the space for sliding 14 and a channel for the connector 15 to access the space for sliding 14. The channel for the connector 15 to access the space for sliding 14 is preferably shaped to closely fit the outer peripheral wall of the connector 15. In this embodiment, the opening of the fixed rod 5 is preferably shaped to closely fit the outer peripheral wall of the connector 15, providing both guidance and positional limitation for the connector 15. Similarly, the first connecting part 3 features a concave structure, with bending portions at both ends provided with hinge holes for installing the first hinge 6. Once the first hinge 6 is installed, an additional opening for the connector 15 to pass through is formed between the first hinge 6 and the tube wall connecting the two bending portions. Preferably, the opening of the first connecting part 3 is also shaped to fit the outer peripheral wall of the connector 15, serving to limit and guide the connector 15, such that when the second retractable rod 16 moves toward the distal end, the openings of both the fixed rod 5 and the first connecting part 3 can restrict the movement of the connector 15 towards the distal end under the pushing force of the second retractable rod 16. This arrangement enables the connector 15 to effectively push the end effector 2 toward the distal end while preventing accumulated deformation of the connector 15 within the second retractable rod 16 and/or the internal space of the rotatable wrist 1. Such deformation, if not prevented, could result from the combined effects of the pushing force of the second retractable rod 16 and the resistance of the end effector 2, potentially rendering the second retractable rod 16 incapable of moving the end effector 2. However, in other embodiments, the fixed rod 5 may also have a solid rod structure, with the second retractable rod 16 arranged parallel to the fixed rod 5. In this configuration, the space for sliding 14 is replaced by a sliding groove in the outer peripheral wall of the rotatable wrist 1, extending along its longitudinal length. The installation end of the end effector 2 is slidably connected to such a sliding groove. To prevent scratches to the human body during operation, tube sleeves must be provided to enclose the rotatable wrist 1 and the installation end of the end effector 2, as well as the second retractable rod 16 and the fixed rod 5.

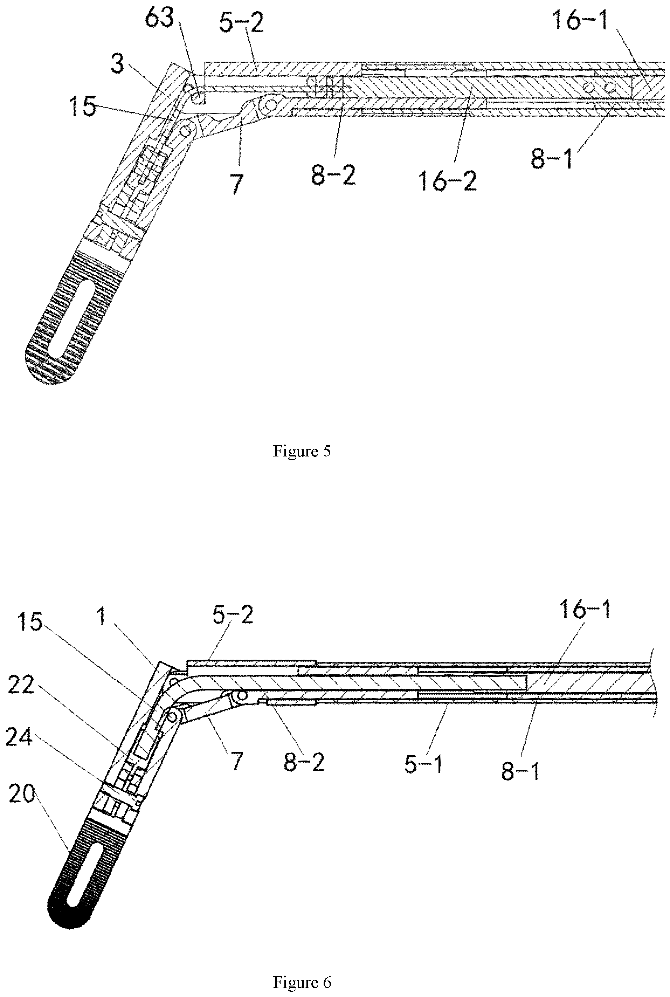

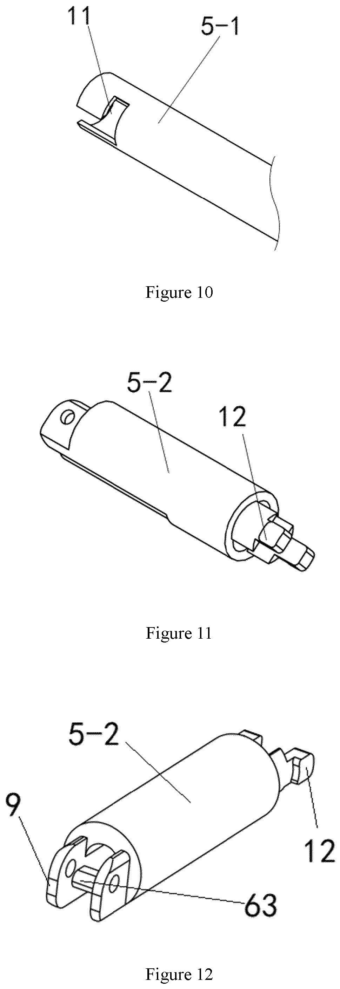

In an embodiment shown in Figs. 5 and 12, the connector 15 does not pass through the additional opening formed between the first hinge 6 and the first connecting part 3. Instead, a limit bar 63 is installed on the first hinge mounting base 9, positioned along a radial direction of the fixed rod 5. A limit space, appropriately sized for the connector 15 to pass through, is formed between the limit bar 63 and the first connecting part 3. In this embodiment, the first connecting part 3 features a concave structure, with bending portions at both ends, each provided with a hinge hole for installing a corresponding first hinge 6. Accordingly, the first hinge 6 does not pass through the concave structure of the first connecting part 3. Instead, one first hinge 6 is arranged at one bending portion, and another first hinge 6 is arranged at another bending portion. The first hinge mounting base 9 is positioned between the two bending portions, and accordingly, the limit bar 63 is also positioned between the two bending portions. In such cases, the limit space serves to guide and restrict the movement of the connector 15. Under the guiding effect of the limit space, the connector 15 transitions from the centerline of the rotatable wrist 1 to the centerline of the fixed rod 5. This alignment ensures that, even when the connector 15 bends and deforms, it remains extension from the centerline of the rotatable wrist 1 onto the centerline of the fixed rod 5, such that the connector 15 will not interfere with the closing of the clamp during bending. This design ensures that the forces involved will not cause coupling phenomena, thereby enabling simultaneous rotation of the rotatable wrist 1 and operation of the clamp. However, in other embodiments, the limit bar 63 may still be present, even if the connector 15 is misaligned with the two centerlines. Alternatively, the connector 15 can be aligned along the two centerlines without the need for the limit bar 63.

In the embodiments shown in Figs. 3, 5, and 6, the first retractable rod 8 has a tubular structure with a second hinge mounting base 17 at its distal end. The second hinge mounting base 17 is hinged to the proximal end of the linkage 7, the first retractable rod 8 is coaxially sleeved over the second retractable rod 16, and the second retractable rod 16 can be driven to move relative to the fixed rod 5. However, in other embodiments, the first retractable rod 8 may also be designed as a solid rod structure, with the second retractable rod 16 and the first retractable rod 8 arranged parallel to each other and both positioned inside the fixed rod 5.

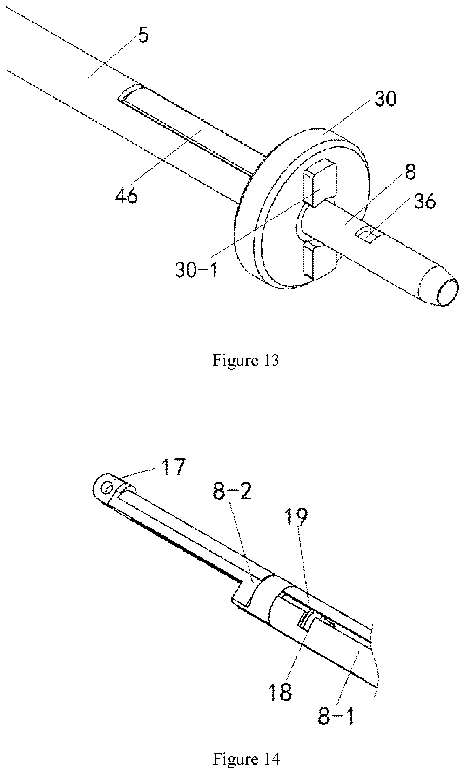

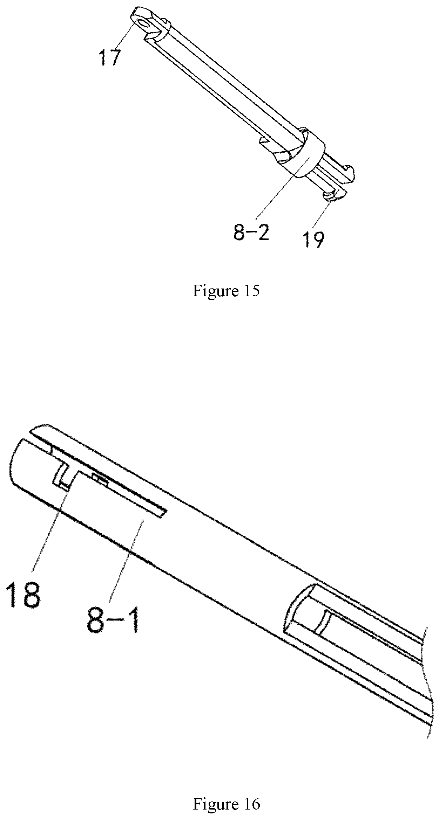

In an embodiment shown in Figs. 14-16, the first retractable rod 8 includes a first retractable rod tube 8-1 and a first retractable rod tube connector 8-2 that are detachably connected. The first retractable rod tube 8-1 features a straight tubular structure, with the second hinge mounting base 17 arranged distally on the first retractable rod tube connector 8-2. Additionally, the first retractable rod tube connector 8-2 is provided with an opening for the second retractable rod 16 or the connector 15 to pass through. To accommodate the arrangement of the second hinge mounting base 17, the first retractable rod 8 is designed as a detachable structure including the first retractable rod tube 8-1 and the first retractable rod tube connector 8-2. This design offers two main advantages: first, it simplifies the production and manufacturing process by enabling separate fabrication of the first retractable rod tube 8-1 with a straight tubular structure and the first retractable rod tube connector 8-2 with a specialized tube shape; second, it facilitates the disassembly and installation of the first retractable rod 8. However, in other embodiments, the first retractable rod 8 may also be an integrated structure, where the first retractable rod tube 8-1 and the first retractable rod tube connector 8-2 are either formed as an integrated unit or fixedly connected.

In an embodiment shown in Figs. 14-17, two bayonets 18 are arranged on the tube wall of the first retractable rod tube 8-1. The first retractable rod tube connector 8-2 is provided at its proximal end with two elastic hooks 19, can extend into the first retractable rod tube 8-1 to engage with one of the two bayonets 18, respectively. As shown in Fig. 17, to facilitate the insertion of the elastic hooks 19 into the first retractable rod tube 8-1 and their engagement with the bayonets 18, two slot channels are provided on the tube wall of the first retractable rod tube 8-1. These channels are arranged radially along the tube and correspond to the two bayonets 18, respectively, with each slot channel communicating with its respective bayonet 18. Additionally, the opening through which the second retractable rod 16 or the connector 15 passes through is positioned between the two elastic hooks 19. However, in other embodiments, the first retractable rod tube 8-1 and the first retractable rod tube connector 8-2 may also utilize a detachable structure incorporating the L-shaped groove 11 and the limit projection 12, as described above. To prevent detachment in use, the rotating thumbwheel 31-2 is omitted. As a result, the first retractable rod 8, the fixed rod 5, the rotatable wrist 1, and the end effector 2 do not have the capability to rotate around the central axis of the fixed rod 5. Alternatively, a plugging socket and a plugging tooth profile, designed to fit together, are provided at the distal end of the first retractable rod tube 8-1 and the proximal end of the first retractable rod tube connector 8-2, respectively. These features ensure circumferential limiting between the first retractable rod tube 8-1 and the first retractable rod tube connector 8-2, allowing them to rotate synchronously when the rotating thumbwheel 31-2 is engaged.

In the embodiment shown in Fig. 3, the second retractable rod 16 includes a second retractable rod body 16-1 and a second retractable rod body connector 16-2. The second retractable rod body 16-1 is positioned inside the first retractable rod tube 8-1, a distal end of the second retractable rod body connector 16-2 is positioned between the second hinge mounting base 17 and the tube wall of the fixed tube connector 5-2 of the fixed rod 5, and a proximal end of the second retractable rod body connector 16-2 passes through the opening on the first retractable rod tube connector 8-2 and is detachably connected to the first retractable rod tube 8-1. The arrangement of the second retractable rod 16, including the detachable second retractable rod body 16-1 and second retractable rod body connector 16-2, is designed to facilitate convenient production, processing, installation, and disassembly. In this embodiment, to prevent interference between the second retractable rod body connector 16-2 and the first retractable rod tube connector 8-2, and to allow the second retractable rod body connector 16-2 to pass through the opening in the first retractable rod tube connector 8-2, the second retractable rod body connector 16-2 is designed in a Z shape. However, in other embodiments, such as the embodiment shown in Fig. 5, the second retractable rod body connector 16-2 may also have a straight configuration. However, in other embodiments, the second retractable rod body 16-1 and the second retractable rod body connector 16-2 may also be designed to be fixedly connected or formed as an integrated structure. For example, in the embodiment where the second retractable rod 16 is arranged parallel to the first retractable rod 8 and the space for sliding 14 is a sliding groove on the outer peripheral wall of the rotatable wrist 1, the second retractable rod 16 can be designed as an integrated structure, with its distal end not requiring a Z shape. Alternatively, in the embodiment shown in Fig. 6, since the connector 15 is a flexible rope, the second retractable rod 16 is designed as a straight rod in an integrated structure, with its distal end not requiring a Z shape.

The connector 15 is an elastic sheet, an elastic rod, or a flexible rope. In the embodiments shown in Figs. 3 and 5, the connector 15 is an elastic sheet or an elastic rod. The structure of the elastic sheet is shown in Fig. 8, with its two ends connected by pins. The elastic sheet is assembled from multiple layers of highly elastic stainless steel sheets. This multi-layered design provides excellent elasticity and enables the elastic sheet to effectively transmit power when subjected to pushing and pulling forces. As a result, it ensures smooth opening and closing of the end effector 2, functioning as a clamp, while also delivering enhanced clamping force. In the embodiment shown in Figs. 6 and 7, the connector 15 is a flexible rope, such as a steel wire rope or a tungsten wire rope. In the embodiment shown in Figs. 6 and 7, the connector 15 extends from the centerline of the rotatable wrist 1 to the centerline of the fixed rod 5.

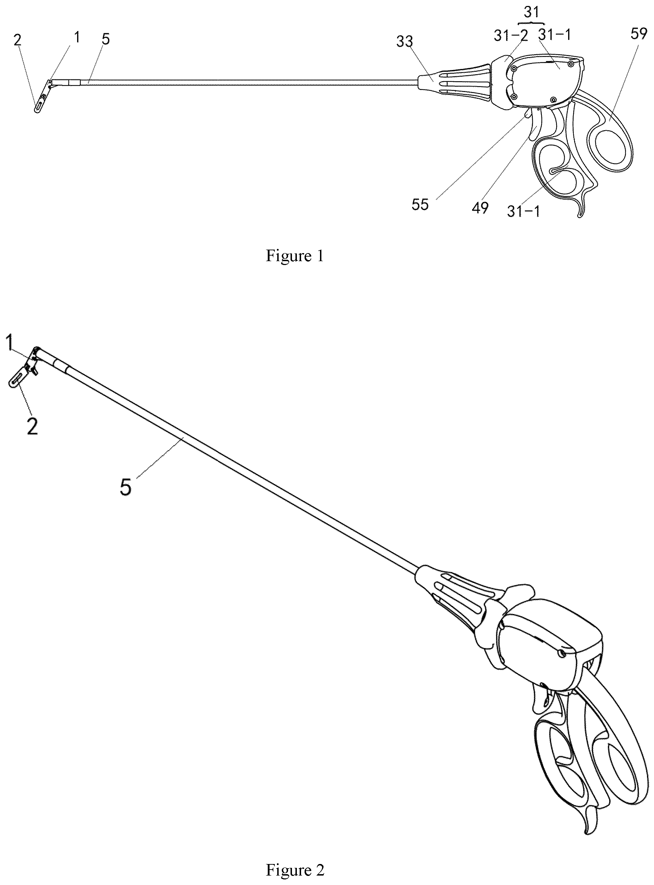

In the embodiment shown in Figs. 2 and 18, the end effector 2 is a clamp that is operated by the second retractable rod 16 to open and close. The clamp includes two claws 20, a claw mounting base 22, and a limit pin 24. At least one of the claws 20 is provided with an arcuate limit hole 21, and this claw 20 is rotatably connected to the rotatable wrist 1 via a first rotating structure. An axis of rotation of the first rotating structure is perpendicular to a direction of movement of the end effector 2, and the first rotating structure is positioned on the concave curved side of the arcuate limit hole 21. The claw mounting base 22 is provided with a linear limit hole 23 extending along a direction of extension and retraction of the end effector 2, and provided with an abutment for abutting against the clamp 20 provided with the arcuate limit hole 21 on a side of the linear limit hole 23 facing the fixed rod 5, and the claw mounting base 22 is connected to the connector 15. The limit pin 24 is arranged at the rotatable wrist 1 and passes through both the linear limit hole 23 and the arcuate limit hole 21. This arrangement enables the opening and closing of the clamp through the extensible and retractable movement of the second retractable rod 16. The limit pin 24, the linear limit hole 23, and the arcuate limit hole 21 work together to restrict the opening and closing angle of the clamp. In this setup, the guided extensible and retractable movement of the end effector 2 along the space for sliding 14 primarily serves to facilitate the opening and closing actions of the clamp, rather than to provide fine adjustments to the position of the clamp. In this embodiment, both claws 20 are provided with arcuate limit holes 21 and are individually connected to the rotatable wrist 1 through their respective first rotating structures. One end of the claw mounting base 22, where the linear limit hole 23 is arranged, is positioned between the two claws 20. This configuration allows both of the claws 20 to perform opening, closing, and rotational movements on the claw mounting base 22. Consequently, the clamp can take various forms, such as a mesentery grasping clamp, non-invasive grasping clamp, matching traction clamp, fixed grasping clamp, V-shaped grasping clamp, or pointed-nozzle grasping clamp. **In** such cases, its structure may not include the arcuate limit hole 21 or the first rotating structure. However, in other embodiments, only one claw 20 may be designed to perform opening, closing, and rotational movements, while the other claw 20 is fixedly connected to the claw mounting base 22 or integrated with it as a single structure. This configuration allows the clamp to function as a type of stomach grasping clamp, single-action mouse-tooth grasping clamp, or the like. However, in other embodiments, the end effector 2 may also be a component such as scissors, a cutter, an electric knife, an electric hook, or the like.

In the embodiment shown in Fig. 18, the distal end of the rotatable wrist 1 is provided with an open-close groove 25 for the opening and closing movement of the claws 20. The first rotating structure includes a first rotating groove 26, arranged on the side wall of the open-close groove 25, and a first rotating protrusion 27, arranged on the claw 20 and designed to fit into the first rotating groove 26. The first rotating protrusion 27 is positioned on a concave curved side of the arcuate limit hole 21. The first rotating structure in this embodiment enables easy disassembly of the claws 20 from the rotatable wrist 1.

In the embodiment shown in Fig. 18, the claw 20 provided with the arcuate limit hole 21 is rotatably connected to the claw mounting base 22 via a second rotating structure, and an axis of rotation of the second rotating structure is perpendicular to the direction of movement of the end effector 2. The second rotating structure is positioned on a side of the arcuate limit hole 21 that is different from the concave curved side. The arrangement of the second rotating structure ensures the stability of the claw mounting base 22 and the claw 20 during their opening and closing rotation. When both claws 20 are capable of opening and closing rotation, the end of the claw mounting base 22 with the linear limit hole 23 is positioned between the two claws 20. It is connected to the claws 20 via two second rotating structures, with each claw 20 corresponding to one second rotating structure. However, in other embodiments, the second rotating structure may be omitted.

In the embodiment shown in Fig. 18, the second rotating structure includes a second rotating protrusion 28 arranged on the claw mounting base 22 and a second rotating groove 29 arranged on the claw 20. The second rotating groove 29 is positioned on a side of the arcuate limit hole 21 opposite to the concave curved side, while the second rotating protrusion 28 is positioned on a side of the linear limit hole 23 in its width direction. The first rotating structure in this embodiment allows for easy disassembly of the claws 20 from the end effector 2.

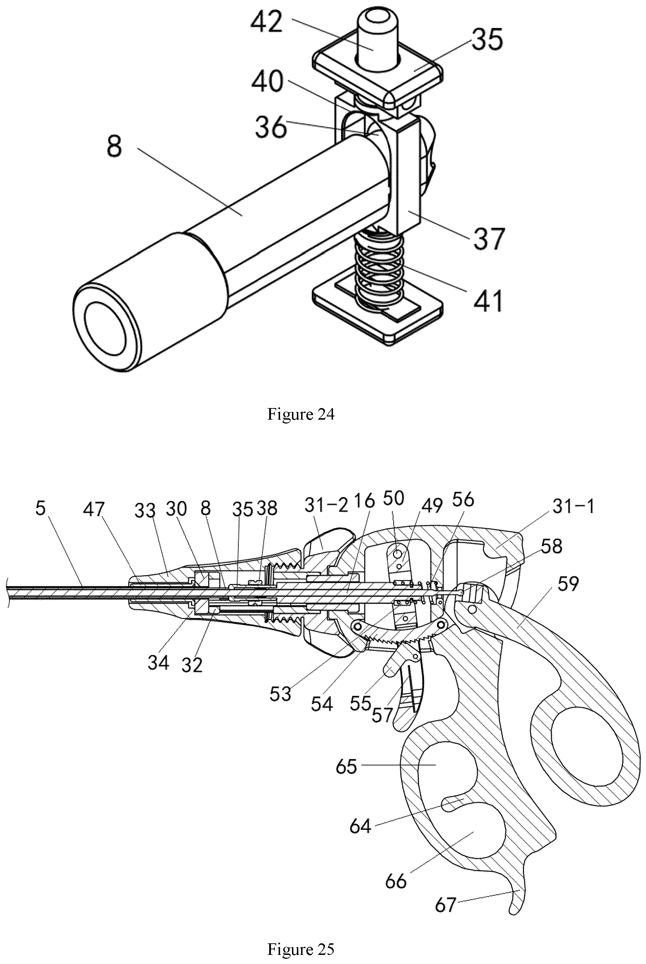

In an embodiment shown in Fig. 25, the minimally invasive surgical instrument assembly further includes a handle 31, an abutter 33, and a quick releaser 35. A first limit protrusion 30 is positioned on the outer peripheral wall of the proximal end of the fixed rod. The handle 31 is provided with a first abutting part 32 that abuts against a proximal end of the first limit protrusion 30. The abutter 33 is detachably connected to the handle 31 and the abutter 33 is provided with a second abutting part 34 that abuts against a distal end of the first limit protrusion 30. The first retractable rod 8 is aligned along the longitudinal length of the fixed rod 5. The quick releaser 35 is arranged on the handle 31 in a retractable moving manner along the longitudinal length of the first retractable rod 8. Also, the quick releaser 35 is detachably connected to the proximal end of the first retractable rod 8 via a quick release structure, allowing the quick releaser 35 to drive the first retractable rod 8 in extensible and retractable motion.

Here, the first limit projection 30 can either be integrally connected to or detachably engaged with the abutter 33. As shown in Fig. 13, an end face of the first limit projection 30 is provided with an eccentric positioning block 30-1, which may be received in a corresponding groove or hole on an end face of the first abutting part 32. This arrangement ensures relative positioning of the first limit projection 30 and the first abutting part 32 in a circumferential direction, preventing their rotational movement relative to each other.

In the mentioned solution, the abutter 33 is configured to abut against the first limit projection 30 of the fixed rod 5, enabling the fixed rod 5 to be securely installed on the handle 31. Moreover, the quick releaser 35, which can connect to the first retractable rod 8, is arranged on the handle 31, allowing the first retractable rod 8 to be installed on the handle 31 as well. Since the abutter 33 is detachably connected to the handle 31 and the quick releaser 35 is connected to the first retractable rod 8 via a quick release structure, the fixed rod 5 and the first retractable rod 8 can be quickly and simply disassembled from the handle 31. This design facilitates separate cleaning and disinfection of the rod body and the handle, effectively reducing the space required for these operations and improving the efficiency and effectiveness of cleaning and disinfection.

The quick release structure includes a catching groove 36, a bayonet 37, and a driving mechanism. The catching groove 36 is arranged on the outer peripheral wall of the proximal end of the first retractable rod 8. The bayonet 37 is arranged at a distal end of the quick releaser 35 and can move between an engaged position, where it is connected to the catching groove 36, and a disengaged position, where it is disconnected from the catching groove 36. The driving mechanism arranged on the quick releaser 35 is capable of driving the bayonet 37 between these two positions, namely the engaged position and the disengaged position. This arrangement allows for quick connection and disconnection between the quick releaser 35 and the first retractable rod 8 by operating the driving mechanism. However, in other embodiments, the quick release structure may also include a slot arranged at a distal end face of the quick releaser 35, a plug and a pin arranged at a proximal end face of the first retractable rod 8, where the slot designed to fit the plug, and both a slot wall of the slot and the plug are provided with pin holes extending radially along the first retractable rod 8, such that the pin can detachably pass through both the slot and the plug.

In the minimally invasive surgical instrument assembly provided by the disclosure, two different types of structures can be adopted for the quick releaser 35, with three implementations provided for the two types of quick releasers 35.

A first type of quick releaser 35 has a rod shape extending along the longitudinal length of the first retractable rod 8, and the bayonet 37 has a hook structure arranged on the quick releaser 35 in a radially movable manner. The driving mechanism includes a collar 38 sleeved over the quick releaser 35. The collar 38 can be driven to move along the longitudinal length of the quick releaser 35. In its distal end position, an inner peripheral wall of the collar 38 at least partially presses against the bayonet 37, holding it in the engaged position. In its proximal end position, the collar 38 is disengaged from the bayonet 37, allowing the bayonet 37 to move to the disengaged position. There may be a single bayonet 37, or two bayonets 37 can be arranged axisymmetrically. When the collar 38 moves distally, the bayonet 37 is embedded into the groove 36 under the pressure exerted by the inner wall of the collar 38. Preferably, the bayonet 37 has a chamfer at its proximal end to prevent it from getting stuck against the collar 38 during operation. When the collar 38 moves proximally, the bayonet 37 can disengage from the groove 36 either manually, by hand-shaking, or automatically, with the assistance of a spring sheet that provides elastic force to the bayonet 37. Two implementations here are provided for this first type of quick releaser 35.

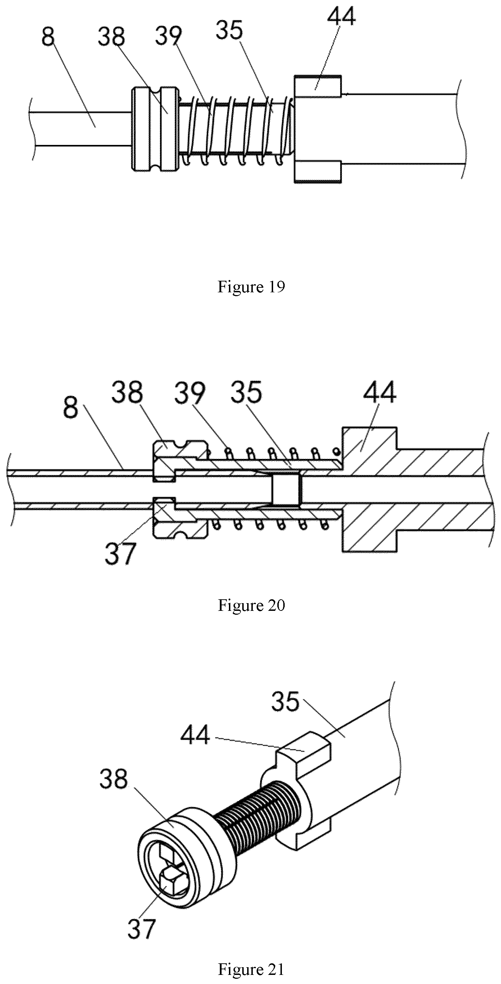

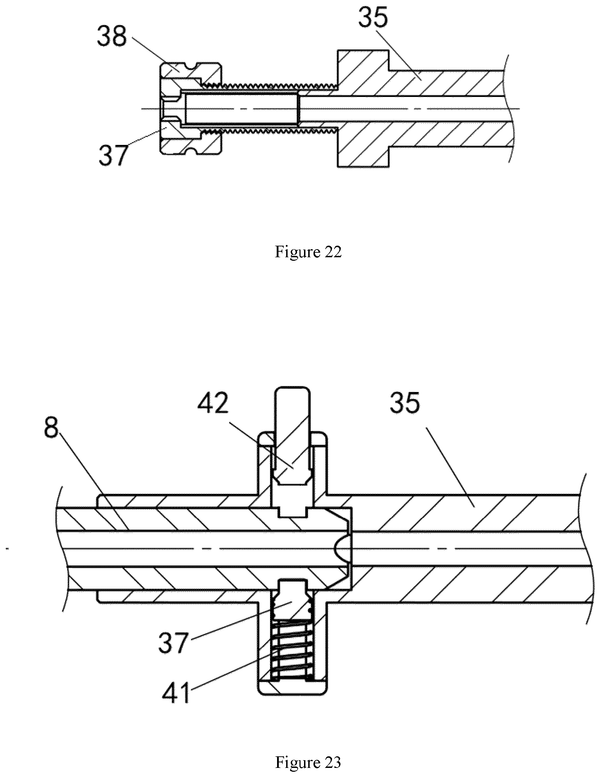

In an implementation of the quick releaser 35, as shown in Figs. 19 and 20, the driving mechanism further includes a first elastic member 39, which is sleeved over an outer peripheral wall of the quick releaser 35. The first elastic member 39 is preferably a spring but may alternatively be a rubber sleeve. The quick releaser 35 is provided with a boss that abuts against the proximal end of the first elastic member 39, and a distal end of the first elastic member 39 abuts against the collar 38, to provide a driving force that moves the collar 38 distally. In this way, the elastic force of the first elastic member 39 ensures that the collar 38 is held in its distal end position. When it is necessary to move the collar 38 proximally, an operator simply needs to push or pull the collar 38 to initiate the movement. In another implementation of the quick releaser 35, as shown in Figs. 21 and 22, the driving mechanism further includes an external thread arranged on the outer peripheral wall of the quick releaser 35, while the inner peripheral wall of the collar 38 is provided with an internal thread that fits the external thread. The movement of the collar 38 is achieved through rotation of the collar 38 by the operator.

A second type of quick releaser 35, shown in Fig. 23 and Fig. 24, involves a further implementation of the quick releaser 35. In this design, the bayonet 37 is arranged at the distal end of the quick releaser 35 in a manner that it is able to move along a direction perpendicular to the length of the first retractable rod 8. The bayonet 37 is designed as a ring with a strip-shaped through-hole 40. The driving mechanism includes a second elastic member 41 and a pressing member 42, which are arranged on opposite sides of the bayonet 37. The second elastic member 41 is preferably a spring, but may alternatively be a rubber cylinder or a sphere. The second elastic member 41 provides a driving force that moves the bayonet 37 towards the pressing member 42, allowing the bayonet 37 to be partially engaged into the catching groove 36. The pressing member 42 can then drive the bayonet 37 to move in an opposite direction, enabling the proximal end of the first retractable rod 8 to enter or exit the strip-shaped through-hole 40. An elastic force of the second elastic member 41 keeps the bayonet 37 in the engaged position. When it is necessary to move the bayonet 37 to the disengaged position, the operator can press the pressing member 42 to push the bayonet 37 into motion. In this embodiment, the pressing member 42 and the bayonet 37 form an integrated structure. However, in other embodiments, the pressing member 42 and the bayonet 37 may also be separate components, with the pressing member 42 abutting against the bayonet 37 to function together. In this embodiment, the catching groove 36 is an annular groove that surrounds the outer peripheral wall at the proximal end of the first retractable rod 8. However, in other embodiments, the catching groove 36 may also be a groove arranged on a side of the first retractable rod 8 facing the bayonet 37. In this embodiment, the bayonet 37 is designed as a ring that is directly engaged into the catching groove 36. However, in other embodiments, a protrusion may also be arranged on the inner peripheral wall of the bayonet 37, extending into the groove 36 for engagement.

As shown in Figs. 25, 27, 19, and 21, the handle 31 includes a handle body 31-1 and a rotating thumbwheel 31-2 that is rotatably arranged on the handle body 31-1. The rotating thumbwheel 31-2 is provided with a through hole located along its axis of rotation. Additionally, the rotating thumbwheel 31-2 is provided with a first strip-shaped limit groove 43, which extends along its axis of rotation and communicates with the through hole. The quick releaser 35 is rotatably connected at its proximal end to the handle body 31-1. Preferably, the proximal end of the quick releaser 35 is shaft-shaped, and a corresponding hinge hole is arranged on the handle body 31-1. The proximal end of the quick releaser 35 is then rotatably inserted into the hinge hole, enabling a detachable connection between the quick releaser 35 and the handle body 31-1. The distal end of the quick releaser 35 passes through the through hole of the rotating thumbwheel 31-2. A first circumferential limit boss 44 is arranged on the outer peripheral wall of the distal end of the quick releaser 35. This first circumferential limit boss 44 is designed to fit into the first strip-shaped limit groove 43 and can move along its longitudinal length. Two side walls of the first strip-shaped limit groove 43, arranged in a circumferential direction of the rotating thumbwheel 31-2, slideably abut against the first circumferential limit boss 44. Through the arrangement of the first strip-shaped limit groove 43 and the first circumferential limit boss 44, the rotating thumbwheel 31-2 can drive both the quick releaser 35 and the first retractable rod 8 to rotate around a central axis of the first retractable rod 8. Additionally, since the first strip-shaped limit groove 43 is shaped in such a way that it will not impede the extensible and retractable movement of the quick releaser 35 and the first retractable rod 8 along the longitudinal length of the first retractable rod 8. As a result, the end effector 2 can rotate around the first hinge 6 relative to the fixed rod 5 and also rotate around the central axis of the first retractable rod 8 by means of the linkage 7 and the rotatable wrist 1. To facilitate the access of the first circumferential limit boss 44 into the first strip-shaped limit groove 43, the portion of the rotating thumbwheel 31-2 where the strip-shaped limit groove 43 is arranged is made of an elastically deformable material, such as plastic, thereby achieving quick installation and detachment of the rotating thumbwheel 31-2 and the quick releaser 35. However, in other embodiments, the rotating thumbwheel 31-2 may be omitted, and only the handle body 31-1 is provided. In this case, the quick releaser 35 and the first retractable rod 8 can move along the longitudinal length of the first retractable rod 8 but cannot rotate around its central axis.

In the embodiment shown in Fig. 25, the handle body 31-1 is provided with a ringshaped gripping portion, and an inner peripheral wall of the gripping portion is provided with a finger clamping projection 64. The finger clamping projection 64 divides the internal space of the gripping portion into a top space 65 adapted for a human middle finger, and a bottom space 66 adapted for a human ring finger. This configuration allows the handle body 31-1 to be moved by the middle finger and the ring finger. The purpose of the finger clamping projection 64 is to enable the middle and ring fingers to securely clamp it, thereby providing a more stable and controlled movement of the handle body 31-1. A finger laying projection 67 is arranged on an outer peripheral wall of the gripping portion, and a distal end face of the finger laying projection 67 is designed to have an arc shape adapted for comfortably resting a human little finger. However, in other embodiments, the handle body 31-1 may also be designed with other existing structures, such as being provided with a grip.

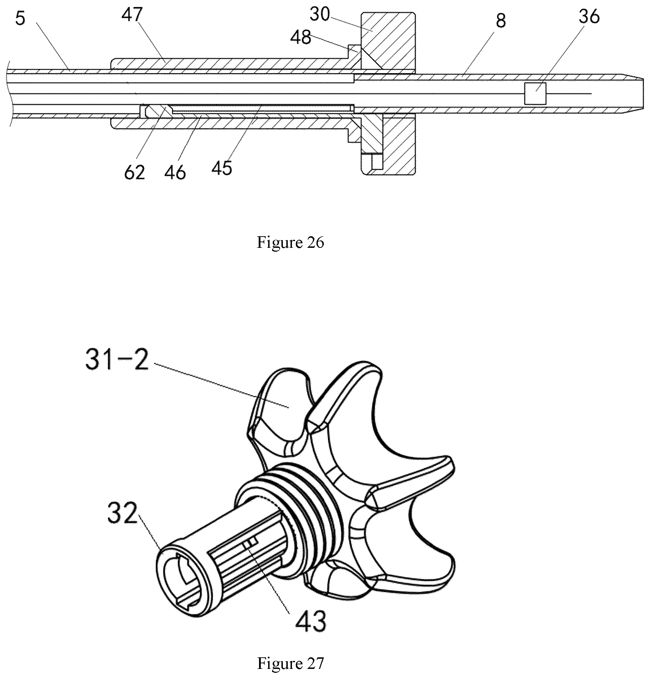

In an embodiment shown in Fig. 26, the fixed rod 5 has a tubular structure that is sleeved over the first retractable rod 8, with the proximal end of the first retractable rod 8 extending out of the fixed rod 5. The proximal end of the fixed rod 5 is provided with an installation groove, while an outer peripheral wall of the first retractable rod 8 is provided with a second strip-shaped limit groove 45 that extends along its longitudinal length. The minimally invasive surgical instrument assembly further includes a circumferential limit member 46, which is designed to be installed in the installation groove of the fixed rod 5. The circumferential limit member 46 is provided with a second circumferential limit boss 62, which is intended to be embedded in the second strip-shaped limit groove 45. The second circumferential limit boss 62 can move within the second strip-shaped limit groove 45 along the longitudinal length of the first retractable rod 8, and the two side walls of the second strip-shaped limit groove 45, positioned in the circumferential direction of the first retractable rod 8, slideably abut against the second circumferential limit boss 62. The arrangement of the circumferential limit member 46, the second strip-shaped limit groove 45, and the second circumferential limit boss 62 enables the fixed rod 5 and the first retractable rod 8 to rotate synchronously around the central axis of the first retractable rod 8. This configuration also allows the first retractable rod 8 to move retractably along the longitudinal length of the fixed rod 5 relative to the fixed rod 5. As a result, when the rotating thumbwheel 31-2 is rotated, both the fixed rod 5 and the first retractable rod 8 rotate simultaneously with the rotating thumbwheel 31-2. However, in other embodiments, the circumferential limit member 46, the second strip-shaped limit groove 45, and the second circumferential limit boss 62 may be omitted if the rotating thumbwheel 31-2 is not included in the design. However, in other embodiments, the fixed rod 5 may also be detachably connected to the rotating thumbwheel 31-2. In this case, the circumferential limit member 46, the second strip-shaped limit groove 45, and the second circumferential limit boss 62 may be omitted. Instead, the fixed rod 5 can be rotated around the central axis of the first retractable rod 8 through the driving force provided by the rotating thumbwheel 31-2. However, in other embodiments, the fixed rod 5 may also have a solid rod body structure, and the fixed rod 5 is arranged in parallel to the first retractable rod 8.

To ensure that the circumferential limit member 46 remains securely in the installation groove and will not detach (which would prevent the fixed rod 5 and the first retractable rod 8 from rotating synchronously), in the embodiments shown in Figs. 25 and 26, the minimally invasive surgical instrument assembly further includes a sleeve 47, which is sleeved over a section of the fixed rod 5 where the installation groove is located, with an inner peripheral wall of the sleeve 47 abutting against the circumferential limit member 46, and an outer peripheral wall of the sleeve 47 provided with a second limit projection 48. The rotating thumbwheel 31-2 is provided with an external threaded section around its axis of rotation, and the first abutting part 32 is formed at the distal end of the rotating thumbwheel 31-2, provided with the first strip-shaped limit groove 43. The abutter 33 is provided with a holed path for the first retractable rod 8 and the fixed rod 5 to pass through. A proximal end of the holed path is provided with an internal threaded section that is connected to the external threaded section of the rotating thumbwheel 31-2, allowing the rotating thumbwheel 31-2 to be detachably connected to the abutter 33, which enables the detachable connection between the handle 31 and the abutter 33. The middle of the hole contains a receiving section, designed to accommodate the first abutting part 32, the distal end of the quick releaser 35, and the first limit projection 30. An abutting section, which is connected to the outer peripheral wall of the sleeve 47 in an abutting manner, is arranged at a distal end of the holed path. Two hole steps are formed between the abutting section and the receiving section. One of these hole steps forms the second abutting part 34, to install the fixed rod 5 on the handle 31. The other of the hole steps abuts against a distal end of the second limit projection 48. A proximal end of the second limit projection 48 abuts against the first limit projection 30 to limit the position of the sleeve 47 on the fixed rod 5, preventing the sleeve 47 from sliding along the fixed rod 5. This design ensures that the sleeve 47 does not detach from the circumferential limit piece 46, as such sliding would cause the circumferential limit piece 46 to be detached from the installation groove. Meanwhile, the outer peripheral wall of the sleeve 47 is rotatably connected to an inner peripheral wall of the holed path in the abutter 33 at its distal end, which will prevent the inner peripheral wall of the holed path from directly abutting against the circumferential limit piece 46, thereby reducing the risk of wear. The holed path in the abutter 33 also provides additional protection for the quick release structure, preventing misoperations that could cause the first retractable rod 8 to become detached from the quick releaser 35. However, in other embodiments, if the rotating thumbwheel 31-2 is not included, the abutter 33 may be arranged in an L-shape, with one end arranged along the longitudinal length of the first retractable rod 8 and connected to the handle body 31-1, and the other end arranged along the radial direction of the first retractable rod 8. The first limit projection 30 would be sandwiched between the other end of the abutter 33 and the handle body 31-1. However, in other embodiments, the sleeve 47 may also be sleeved over the fixed rod 5 by using a threaded structure, eliminating the need for introducing the second limit projection 48.

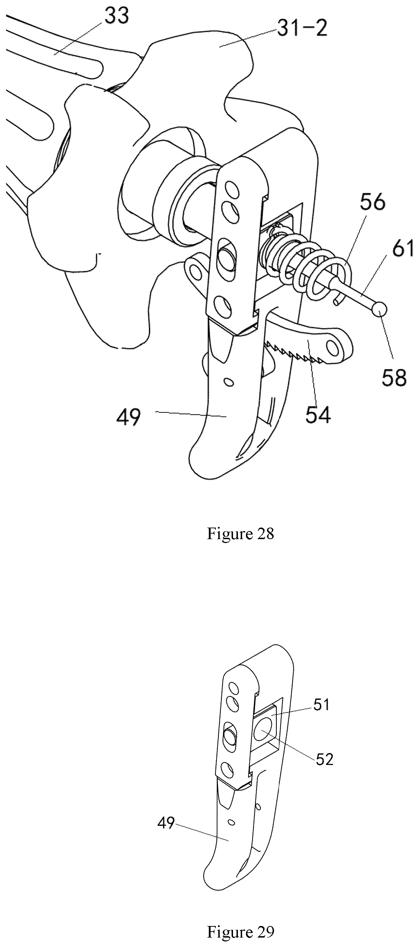

As shown in Figs. 25, 28, and 29, the minimally invasive surgical instrument assembly further includes a first trigger 49 and a rotating mounting base 51. The first trigger 49 is rotatably hinged to the handle 31 via a first trigger hinge 50. In this embodiment, the first trigger hinge 50 is located at the non-handheld end of the first trigger 49. However, in other embodiments, the first trigger hinge 50 may also be arranged in the middle of the first trigger 49. The rotating mounting base 51 is rotatably arranged at the non-handheld end of the first trigger 49, with an axis of rotation of the rotating mounting base 51 being parallel to the first trigger hinge 50. In this embodiment, protruding axial protrusions are provided on both sides of the rotating mounting base 51, and these axial protrusions are rotatably connected to the first trigger 49. The rotating mounting base 51 is provided with an installation hole 52, with an axis of the installation hole 52 being perpendicular to the first trigger hinge 50. The quick releaser 35 is arranged inside the installation hole 52, allowing it to rotate around the axis of the installation hole 52. As a result, when the first trigger 49 is rotated around the first trigger hinge 50, the quick releaser 35 can be driven to move retractably along the longitudinal length of the first retractable rod 8, thereby enabling the quick releaser 35 to rotate around the central axis of the first retractable rod 8 and to move retractably along its longitudinal direction. However, in other embodiments, when the rotating thumbwheel 31-2 is not included, the rotating mounting base 51 may be omitted. In this case, the proximal end of the quick releaser 35 is directly hinged to the first trigger 49, thereby allowing the quick releaser 35 and the first retractable rod 8 to be driven by the first trigger 49, enabling them to move along the longitudinal length of the first retractable rod 8.

As shown in Fig. 25, an inner peripheral wall of the installation hole 52 is fixedly provided with a rotatable connecting sleeve 53, which is rotatably sleeved at the proximal end of the quick releaser 35, avoiding stress concentration at the proximal end of the quick releaser 35 when the first trigger 49 is rotated. This design will minimize the risk of damage to the proximal end of the quick releaser 35. However, in other embodiments, when the installation hole 52 has a sufficient length along its axial direction, the rotatable connecting sleeve 53 may be omitted.

In the embodiment shown in Fig. 25, a distal end face of the handheld end of the first trigger 49 is shaped with an arc that is adapted for comfortably holding a human index finger. The first trigger 49 is positioned on a distal side of the gripping portion of the handle body 31-1. However, in other embodiments, the first trigger 49 may also be constructed in other existing structures.

In the embodiment shown in Fig. 25, the minimally invasive surgical instrument assembly further includes an arcuate ratchet sheet 54 and a position locker 55. The arcuate ratchet sheet 54 is arranged on the handle 31, surrounding the first trigger hinge 50, and is provided with a plurality of ratchet slots along its radial direction. The position locker 55 is provided with a toothed portion for being embedded into the ratchet slots, and it is rotatably arranged on the first trigger 49, such that the position locker 55 has a locked position, where the toothed portion is embedded in the ratchet slots, and an active position, where the toothed portion is detached from the grooves. When the position locker 55 is in the locked position, the first trigger 49 can only move unidirectionally along an arc direction of the arcuate ratchet sheet 54. In Fig. 25, the unidirectional movement is illustrated in a counterclockwise direction. By changing the arrangement direction of the arcuate ratchet sheet 54, the unidirectional direction of movement can be adjusted. The arrangement of the arcuate ratchet sheet 54 and the position locker 55 allows for the position locking of the first retractable rod 8 in its longitudinal position when the first trigger 49 is not pulled. The position locker 55, when in the locking position, limits the movement of the first retractable rod 8. This configuration enables the adjustment of the end effector 2 to rotate around the first hinge 6 relative to the fixed rod 5, with the rotation angle limitation for the end effector 2 being achieved by the position locker 55. In other words, when the position locker 55 is in the locked position, the first trigger 49 can only move unidirectionally along the arc direction of the arcuate ratchet sheet 54, which restricts movement in an opposite direction. To allow movement in the opposite direction, the position locker 55 must be moved to the active position. In this way, the position locker 55 functions as a limit switch. However, in other embodiments, the arcuate ratchet sheet 54 and position locker 55 may be omitted. In this case, an operator would need to continuously control the position of the first trigger 49 to limit and adjust the movement of the first retractable rod 8, thereby achieving the limitation and adjustment of the rotation angle of the end effector 2.

In the embodiment shown in Fig. 25, the minimally invasive surgical instrument assembly further includes a third elastic member 56, which is arranged along the longitudinal length of the first retractable rod 8. In this embodiment, the handle 31 is connected to the rotating mounting base 51 via the third elastic member 56, and the third elastic member 56 provides a force to the first trigger 49 in a direction opposite to the unidirectional direction of movement. In this embodiment, the third elastic member 56 provides a force that drives the first trigger 49 towards the distal end, meaning that the third elastic member 56 plays a role in resetting the first trigger 49. When the position locker 55 is moved to the active position, the third elastic member 56 automatically drives the first trigger 49 to reset, which in turn eliminates the rotation angle of the end effector 2 and resets it to the central axis of the first retractable rod 8. Once the operator completes the operation with the end effector 2, he/she only needs to move the position locker 55 to the active position, then the minimally invasive surgical instrument assembly can be removed from the human body without having to manually operate the first trigger 49 to reset the end effector 2. However, in other embodiments, it is also possible to connect the handle 31 to the proximal end of the first retractable rod 8 via the third elastic member 56, or alternatively, connect the handle 31 to the first trigger 49 via the third elastic member 56. Certainly, in other embodiments, the third elastic member 56 may be omitted. In this embodiment, the third elastic member 56 is a spring that is sleeved over the proximal end of the quick releaser 35 and the second retractable rod 16, as described below. However, in other embodiments, the third elastic member 56 may also be a rubber collar or a rubber rod. Additionally, the third elastic member 56 may not be sleeved over the proximal end of the quick releaser 35 and the second retractable rod 16, but instead may be directly connected to the proximal end of the quick releaser 35 and the handle 31. In such configurations, the third elastic member 56 could also be arranged at a certain angle with the axial direction of the first retractable rod 8.

In the embodiment shown in Fig. 25, the minimally invasive surgical instrument assembly further includes a fourth elastic member 57, which is arranged on the first trigger 49. The fourth elastic member 57 exerts pressure on the position locker 55, which is located at the locked position, to push the toothed portion of the position locker 55 towards the ratchet slots. This ensures that the toothed portion remains securely engaged into the ratchet slots, preventing detachment that could otherwise affect the surgical operation when the position locker 55 is limiting the first trigger 49. In this embodiment, an elastic sheet is adopted as the fourth elastic member 57. However, in other embodiments, a spring may also be adopted as the fourth elastic member 57, or the fourth elastic member 57 may be omitted entirely.

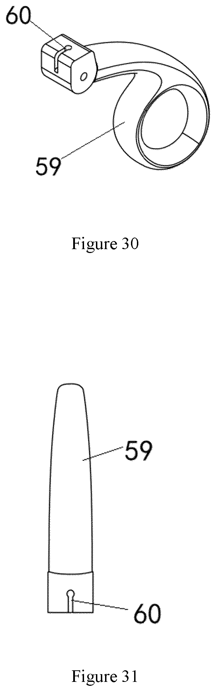

As shown in Figs. 25, 30, and 31, the minimally invasive surgical instrument assembly further includes the second retractable rod 16 and a second trigger 59. The second retractable rod 16 is arranged along the longitudinal length of the fixed rod 5, with a bulbous tip 58 provided at the proximal end of the second retractable rod 16. The second trigger 59 is rotatably hinged to the handle 31 via a second trigger hinge. A sector-shaped sliding groove 60 is arranged at a non-handheld end of the second trigger 59, with a plane formed by the sector-shaped sliding groove 60 being perpendicular to the second trigger hinge. A curved bottom of the sector-shaped sliding groove 60 is shaped to limit the bulbous tip 58, allowing it to slide only along its arc direction. In this embodiment, the second retractable rod 16 is further integrated into the assembly. As can be understood from the following description, the second retractable rod 16 is used to adjust the position of the end effector 2 along the longitudinal length of the fixed rod 5, allowing for fine adjustment of position of the end effector 2 or enabling the opening and closing operation of the clamp as the end effector 2. The second trigger 59 is designed to drive the second retractable rod 16 to move retractably along the longitudinal length of the fixed rod 5, which in turn drives the end effector 2. The bulbous tip 58 is arranged at the proximal end of the second retractable rod 16, while the sector-shaped sliding groove 60 is arranged at the non-handheld end of the second trigger 59, such that a detachable connection between the second trigger 59 and the second retractable rod 16 can be achieved. By rotating the second trigger 59, the proximal end of the second retractable rod 16 can either be detached from or enter into the sector-shaped sliding groove 60. Meanwhile, the curved bottom of the sector-shaped sliding groove 60 is shaped for limiting the bulbous tip 58, allowing the bulbous tip 58 to slide only along its arc direction. As a result, when the second trigger 59 is pulled, the bulbous tip 58 can be driven to move retractably along the longitudinal length of the fixed rod 5, which in turn drives the second retractable rod 16 to move. However, in other embodiments, if the end effector 2 is fixedly arranged on the rotatable wrist 1, or if the end effector 2 is fixedly arranged on the first retractable rod 8, the second retractable rod 16 and the second trigger 59 may be omitted. In such cases, the first trigger 49 is designed to drive the first retractable rod 8 to move along the longitudinal length of the fixed rod 5, thereby enabling the end effector 2 to move along the longitudinal length of the fixed rod 5, rather than driving the rotatable wrist 1 to rotate around the first hinge 6 relative to the fixed rod 5.

In the embodiment shown in Fig. 25, the second trigger 59 is designed with a circular structure that is adapted for a human thumb to be inserted, and the second trigger 59 is positioned on a proximal side of the gripping portion of the handle body 31-1. However, in other embodiments, the second trigger 59 may also be designed with other existing structures.

In the embodiment shown in Fig. 25, the first retractable rod 8 has a tubular structure that is sleeved over the second retractable rod 16, with the proximal end of the second retractable rod 16 extending from the first retractable rod 8. A small-diameter section 61, with a diameter smaller than the inner tube diameter of the second retractable rod 16, is arranged at the proximal end of the second retractable rod 16. The bulbous tip 58 is arranged at a proximal end of the small-diameter section 61, and the bulbous tip 58 has a diameter less than or equal to the inner diameter of the second retractable rod 16. The small-diameter section 61 is arranged to form the bulbous tip 58, with its diameter less than or equal to the inner diameter of the second retractable rod 16. The diameter of the bulbous tip 58 is less than or equal to the inner diameter of the second retractable rod 16, allowing the proximal end of the second retractable rod 16 to be inserted into the first retractable rod 8, thereby enabling the disassembly of the second retractable rod 16 from the first retractable rod 8. However, in other embodiments, the first retractable rod 8 may also have a solid rod structure, with the second retractable rod 16 arranged parallel to the first retractable rod 8. In this case, the diameter of the bulbous tip 58 would not need to be limited.

Another aspect of the disclosure provides a minimally invasive surgical instrument, which may include the described minimally invasive surgical instrument assembly.

Here, by application of the disclosed minimally invasive surgical instrument assembly, the rotatable wrist 1 can be driven to rotate about the first hinge 6 when the first retractable rod 8 moves retractably along the longitudinal length of the fixed rod 5, thereby enabling the rotation of the end effector 2. Moreover, the disclosed structure features the smaller turning radius compared to existing joint wrist structures, effectively addressing challenges faced by minimally invasive surgical instruments, such as difficulties in rotating the end effector 2 within confined spaces or potential interference with other surgical instruments due to their larger turning radius.

When the minimally invasive surgical instrument is operated, the index finger pulls the first trigger 49, the thumb is hooked in the second trigger 59, and the middle finger, ring finger, and little finger are placed on the handle body 31-1 from top to bottom, respectively. This grip allows for a more ergonomic and convenient operation of the instrument. The second trigger 59 controls the opening and closing of the clamp, which serves as the end effector 2. The first trigger 49 can be hooked backwards to achieve the rotation of the end effector 2 and the rotatable wrist 1 around the first hinge 6, relative to the fixed rod 5. The position locker 55 and the third elastic member 56 allow for the quick and direct return of the end effector 2 and the rotatable wrist 1 from a rotated position to a straight clamp state, where both the end effector 2 and the rotatable wrist 1 are aligned along the central axis of the fixed rod 5. This enables the distal end of the minimally invasive surgical instrument to be quickly and conveniently removed from a small hole on a patient's body surface. Additionally, when rotation of the end effector 2 around the central axis of the fixed rod 5 is required, the rotating thumbwheel 31-2 is rotated by the index finger.

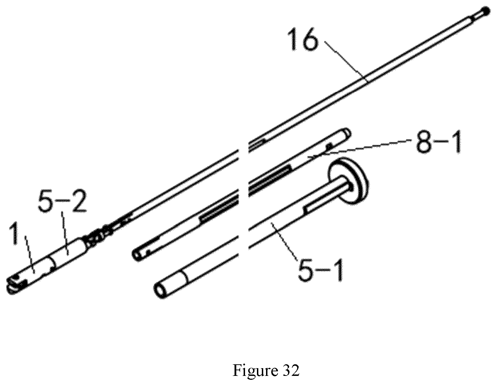

Meanwhile, since the fixed rod 5, the first retractable rod 8, and the second retractable rod 16 are all connected to the handle 31 in a quickly detachable manner, they can be easily and quickly detached from the handle 31 when the minimally invasive surgical instrument needs to be cleaned and disinfected. A disassembled structure is shown in Fig. 32. In such cases, the fixed tube 5-1 is disassembled into independent components, and the first retractable rod tube 8-1 is also disassembled into independent components. Meanwhile, the second retractable rod 16, the fixed tube connector 5-2, the first retractable rod tube connector 8-2, and the rotatable wrist 1 remain connected together. This allows the three parts to be cleaned and disinfected separately, improving the cleaning and disinfection effect while effectively reducing the space required for cleaning and disinfection operations. Certainly, further detachment can be performed in other implementations.

The preferred embodiments of the disclosure have been described in detail above in combination with the accompanying drawings, but the disclosure is not limited thereto. Within the scope of the inventive concept of the disclosure, various simple variations may be made to the solution, including the combination of individual features in any suitable manner. In order to avoid unnecessary repetition, the disclosure will not separately explain the various possible combinations. However, these simple variations and combinations should also be considered as part of the disclosure, and all of them shall fall within the scope of the disclosure.

**List of Reference Numbers**

| | | | |
|---|---|---|---|
| 1 | Rotatable wrist | 32 | First abutting part |
| 2 | End effector | 33 | Abutter |
| 3 | First connecting part | 34 | Second abutting part |
| 4 | Second connecting part | 35 | Quick releaser |
| 5 | Fixed rod | 36 | Catching groove |
| 5-1 | Fixed tube | 37 | Snap-fitter |
| 5-2 | Fixed tube connector | 38 | Collar |
| 6 | First hinge | 39 | First elastic member |
| 7 | Linkage | 40 | Strip-shaped through hole |
| 8 | First retractable rod | 41 | Second elastic member |
| 8-1 | First retractable rod tube | 42 | Pressing member |
| 8-2 | First retractable rod tube connector | 43 | First strip-shaped limit groove |
| 9 | First hinge mounting base | 44 | First circumferential limit boss |
| 10 | First receiving groove | 45 | Second strip-shaped limit groove |
| 11 | L-shaped groove | 46 | Circumferential limiter |
| 12 | Limit protrusion | 47 | Casing tube |
| 13 | Second receiving groove | 48 | Second limit projection |
| 14 | Space for sliding | 49 | First trigger |
| 15 | Connector | 50 | First trigger hinge |
| 16 | Second retractable rod | 51 | Rotating mounting base |
| 16-1 | Second retractable rod body | 52 | Installation hole |
| 16-2 | Second retractable rod body connector | 53 | Rotatable connecting sleeve |
| 17 | Second hinge mounting base | 54 | Arcuate ratchet sheet |
| 18 | Bayonet | 55 | Position locker |
| 19 | Elastic hook | 56 | Third elastic member |
| 20 | Claw | 57 | Fourth elastic member |
| 21 | Arcuate limit hole | 58 | Bulbous tip |
| 22 | Claw mounting base | 59 | Second trigger |
| 23 | Linear limit hole | 60 | Sector-shaped sliding groove |
| 24 | Limit pin | 61 | Small-diameter section |
| 25 | Open-close groove | 62 | Second circumferential limit boss |
| 26 | First rotating groove | 63 | Limit bar |
| 27 | First rotating protrusion | 64 | Finger clamping projection |
| 28 | Second rotating protrusion | 65 | Top space |
| 29 | Second rotating groove | 66 | Bottom space |
| 30 | First limit projection | 67 | Finger laying projection |
| 30-1 | Positioning block | | |
| 31 | Handle | | |
| 31-1 | Handle body | | |
| 31-2 | Rotating thumbwheel | | |

## Claims

1. A minimally invasive surgical instrument assembly, **characterized by** comprising:
a rotatable wrist (1) with a distal end to which an end effector (2) is attachable and a proximal end provided with a first connecting part (3) and a second connecting part (4);
a fixed rod (5) with a distal end rotatably connected to the first connecting part (3) via a first hinge (6);
a linkage (7) with a distal end rotatably hinged to the second connecting part (4); and
a first retractable rod (8) detachably connected to the fixed rod (5), with a distal end rotatably hinged to a proximal end of the linkage (7), the first retractable rod (8) being drivable to move retractably along a longitudinal length of the fixed rod (5), to force the rotatable wrist (1) to rotate around the first hinge (6) relative to the fixed rod (5),
wherein the rotatable wrist (1) is provided with a space for sliding (14); and
wherein the minimally invasive surgical instrument assembly further comprises:
an end effector (2) with an installation end located within the space for sliding (14);
a flexibly deformable connector (15) positioned where the rotatable wrist (1) is connected to the fixed rod (5), with a distal end attached to the installation end of the end effector (2); and
a second retractable rod (16) with a distal end connected to a proximal end of the connector (15), the second retractable rod (16) being drivable to move retractably along the longitudinal length of the fixed rod (5), to force the end effector (2) to move retractably in a direction of extending into or retracting from the space for sliding (14),
wherein the fixed rod (5) has a tubular structure with a first hinge mounting base (9) at its distal end, wherein the second retractable rod (16) is located inside the fixed rod (5), and an opening for the connector (15) to pass through is arranged between the first hinge mounting base (9) and a tube wall of the fixed rod (5), and
wherein the rotatable wrist (1) is internally hollow to form the space for sliding (14) and a channel for the connector (15) to enter the space for sliding (14);
wherein the first hinge mounting base (9) is provided with a limit bar (63), which is arranged radially along the fixed rod (5), and a limit space adapted for the connector (15) to pass through is formed between the limit bar (63) and the first connecting part (3), and/or
wherein the connector (15) extends from a centerline of the rotatable wrist (1) to a centerline of the fixed rod (5);
wherein the connector (15) is an elastic sheet, an elastic rod or a flexible rope, and
wherein the end effector (2) is a clamp that is driven by the second retractable rod (16) to open and close, the clamp comprising:
two claws (20), at least one of which is provided with an arcuate limit hole (21) and is rotatably connected to the rotatable wrist (1) via a first rotating structure, wherein the first rotating structure has an axis of rotation perpendicular to a direction of movement of the end effector (2), and the first rotating structure is positioned on a concave curved side of the arcuate limit hole (21);
a claw mounting base (22) provided with a linear limit hole (23) extending along a direction of extension and retraction of the end effector (2), and provided with an abutment for abutting against the clamp (20) provided with the arcuate limit hole (21) on a side of the linear limit hole (23) facing the fixed rod (5), the claw mounting base (22) being connected to the connector (15); and
a limit pin (24) arranged at the rotatable wrist (1), the limit pin (24) penetrating through the linear limit hole (23) and the arcuate limit hole (21),
wherein the clamp (20) provided with the arcuate limit hole (21) is rotatably connected to the claw mounting base (22) via a second rotating structure, wherein the second rotating structure has an axis of rotation perpendicular to the direction of movement of the end effector (2), and the second rotating structure is positioned on a side of the arcuate limit hole (21) different from the concave curved side of the arcuate limit hole (21).

2. The minimally invasive surgical instrument assembly of claim 1, **characterized in that** the fixed rod (5) has a tubular structure with the first hinge mounting base (9) at its distal end, wherein the first hinge mounting base (9) is hinged to the first connecting part (3), and the tube wall of the fixed rod (5) is provided with a first receiving groove (10) communicating with an internal space of the tubular structure, and wherein the first retractable rod (8) is located inside the fixed rod (5), and the distal end of the first retractable rod (8) is received in the first receiving groove (10) to connect with the proximal end of the linkage (7).

3. The minimally invasive surgical instrument assembly of claim 2, **characterized in that** the fixed rod (5) comprises a fixed tube (5-1) and a fixed tube connector (5-2) that are detachably connected, wherein the fixed tube (5-1) has a straight tubular structure, and the first hinge mounting base (9) and the first receiving groove (10) are arranged distally on the fixed tube connector (5-2).

4. The minimally invasive surgical instrument assembly of claim 3, **characterized in that** an L-shaped groove (11) arranged in a tube wall at a distal end of the fixed tube (5-1) comprises a limit portion extending circumferentially around the fixed tube (5-1) and an access passage extending axially along the fixed tube (5-1), the access passage defining an opening at an end of the fixed tube (5-1), and a radially protruding limit protrusion (12) is arranged on an outer peripheral surface of a proximal end of the fixed tube connector (5-2), the limit protrusion (12) being engageable or disengageable from the limit portion through the access passage, wherein the limit portion is adapted in length to the limit protrusion (12) along the axial direction of the fixed tube (5-1).

5. The minimally invasive surgical instrument assembly of claim 2, **characterized in that** the proximal end of the rotatable wrist (1) includes a tube wall with an axially protruded portion forming the first connecting part (3) and a non-protruded portion forming the second connecting part (4).

6. The minimally invasive surgical instrument assembly of claim 1, **characterized in that** the first retractable rod (8) has a tubular structure with a second hinge mounting base (17) at its distal end, wherein the second hinge mounting base (17) is hinged to the proximal end of the linkage (7), the first retractable rod (8) is coaxially sleeved outside the second retractable rod (16), and the second retractable rod (16) is drivable to move retractably relative to the fixed rod (5).

7. The minimally invasive surgical instrument assembly of claim 6, **characterized in that** the first retractable rod (8) comprises a first retractable rod tube (8-1) and a first retractable rod tube connector (8-2) that are detachably connected, wherein the first retractable rod tube (8-1) has a straight tubular structure, the second hinge mounting base (17) is arranged distally on the first retractable rod tube connector (8-2), and the first retractable rod tube connector (8-2) is further provided with an opening for the second retractable rod (16) or the connector (15) to pass through.

8. The minimally invasive surgical instrument assembly of claim 7, **characterized in that** the first retractable rod tube (8-1) has a tube wall provided with two bayonets (18), and the first retractable rod tube connector (8-2) has a proximal end provided with two elastic hooks (19), each capable of extending into the first retractable rod tube (8-1) to engage with one of the two bayonets (18) respectively, wherein the opening for the second retractable rod (16) or the connector (15) to pass through is positioned between the two elastic hooks (19).

9. The minimally invasive surgical instrument assembly of claim 7, **characterized in that** the second retractable rod (16) comprises a second retractable rod body (16-1) located inside the first retractable rod tube (8-1) and a second retractable rod body connector (16-2), with its distal end positioned between the second hinge mounting base (17) and a tube wall of the fixed tube connector (5-2) of the fixed rod (5), and its proximal end passing through the opening in the first retractable rod tube connector (8-2) and being detachably connected to the first retractable rod tube (8-1).
